# EUROPEAN PATENT SPECIFICATION

(11) **EP 4 373 398 B1**
(45) Date of publication and mention of the grant of the patent: **28.10.2026**
(21) Application number: 22846829.4
(22) Date of filing: 21.07.2022
(51) Int. Cl.: A61B 5/103, A61B 5/107, A61B 8/00, A61B 8/08, A61B 5/08

(54) **ENERGY WAVE REFRACTION AND METHODOLOGY TO DETECT GASTRIC DISTENTION AND PREVENT ASPIRATION**
ENERGIEWELLENREFRAKTION UND METHODOLOGIE ZUR DETEKTION VON MAGENVERSCHRÄNKUNG UND ZUR ASPIRATIONSVERHINDERUNG
RÉFRACTION D'ONDE D'ÉNERGIE ET MÉTHODOLOGIE POUR DÉTECTER UNE DISTENSION GASTRIQUE ET ÉVITER UNE ASPIRATION

(30) Priority: 21.07.2021 US 202163224339 P
(43) Date of publication of application: 29.05.2024
(60) Divisional application: 26206174.0
(73) Proprietor: Baylor College of Medicine, Houston, TX 77030 (US)
(72) Inventor: ROSENGART, Todd, Houston, Texas 77030 (US); MORTUS, Jared, Houston, Texas 77030 (US)
(74) Representative: J A Kemp LLP
(86) International application number: PCT/US2022/073987
(87) International publication number: WO 2023/004378

(56) References cited:
- US-A1- 2016 331 298
- US-A1- 2016 331 298
- US-A1- 2017 169 725
- US-A1- 2018 078 195
- US-A1- 2019 142 324
- US-A1- 2019 209 067
- HUH JIMI ET AL: "CT gastrography for volumetric measurement of remnant stomach after distal gastrectomy: a feasibility study", ABDOMINAL RADIOLOGY, SPRINGER US, NEW YORK, vol. 41, no. 10, 1 June 2016 (2016-06-01), pages 1899 - 1905, XP036052736, ISSN: 2366-004X, [retrieved on 20160601], DOI: 10.1007/S00261-016-0792-X
- SHARMA VIBHU, DEEPTHI GUDIVADA, RENAUD GUERET, JOHN BAILITZ : "Ultrasound-assessed gastric antral area correlates with aspirated tube feed volume in enterally fed critically ill patients", NUTRITION IN CLINICAL PRACTICE, vol. 32, no. 2, pages 206 - 211, XP093027716, DOI: 10.1177/0884533616681530
- VAN DE PUTTE P., PERLAS A.: "Ultrasound assessment of gastric content and volume", BRITISH JOURNAL OF ANAESTHESIA, vol. 113, no. 1, 1 July 2014 (2014-07-01), pages 12 - 22, XP093027718, ISSN: 0007-0912, DOI: 10.1093/bja/aeu151

## Description

### I. Technical Field

Embodiments of the disclosure relate at least to the fields of medicine, medical devices, and physiology.

### II. Background

While commonly considered as an anesthetic complication, pulmonary aspiration of gastric content is one of the leading causes of morbidity and mortality in hospitalized patients. Aspiration can be identified as occurring in as many as 30% of critically ill patients and leading to mortality in at least 20% to 30% of patients with aspiration pneumonia (1, 2). Mortality may exceed 70% in-patients with aspiration pneumonia with mortality risk factors (1, 2). Aspiration pneumonia may occur in nearly half of patients with aspiration events, and is associated with prolonged ventilator support, intensive care unit length of stay and hospital length of stay.

Aspiration is typically associated with gastric distension and impaired mental status, often due to hospital-related patient immobilization, sedation or other risk factors for reduced consciousness and/or bowel motility. Defined as the entry of liquid or solid material into the trachea and lungs, aspiration occurs when patients without sufficient laryngeal protective reflexes passively or actively regurgitate gastric contents. In almost all cases, increased gastric distension in the patient with impaired consciousness leads to increased gastric pressure and regurgitation of gastric contents that overwhelms the normal protective functional barriers of the esophageal sphincter and laryngeal competency mechanisms, respectively. Regurgitation, vomiting and aspiration may also occur quite unexpectedly in association with anesthesia and may have serious consequences.

Pulmonary syndromes resulting from aspiration range from those manifested by mild symptoms, such as hypoxia, to complete respiratory failure and acute respiratory distress syndrome (ARDS), and even cardiopulmonary collapse and death. The types of pulmonary syndromes include acid-associated pneumonitis (most common), particle-associated aspiration (e.g., airway obstruction), or bacterial infection, with subsequent development of lung abscess, exogenous lipoid pneumonia, chronic interstitial fibrosis, and *Mycobacterium fortuitum* pneumonia. Which of these syndromes develops depends on the composition and volume of the aspirate.

In all cases, the key to minimizing the impact of aspiration is to prevent it from happening. Aspiration mitigation is usually based on assuring patients are exposed to sufficient fasting times prior to periods of impaired consciousness, such as prior to anesthetic-induced degradation of consciousness. Assurance of fasting is however not applicable for patients in urgent or emergent situations requiring induction of anesthetic sedation.

Importantly, in addition to the incidence of peri-operative aspiration, an even more confounding challenge is aspiration that occurs in the hospitalized patient who is obtunded and/or otherwise without normally functioning GI activity. In such circumstances, fasting for prolonged periods is neither desirable nor feasible in hospitalized patients, as it will induce malnutrition. In such patients, confirmation and maintenance of normal gastrointestinal function and gastric emptying is an important consideration in preventing aspiration.

An example of an aspiration risk patient would be one with sepsis and diminished bowel activity with some degree of diminished consciousness (intrinsic or medically-induced) or the post-operative patient with similar impairments. In this context, while prophylactic measures noted below may be relevant to the immediate peri-op period where more intensive acute monitoring, assessment and intervention is feasible, such measures are not conducive to aspiration treatment for patients in this latter category where prolonged (days-long) monitoring would be required.

Preemptive nasogastric (NG) tube placement has been proposed as an option for reducing aspiration risk in patients with aspiration risk. However, evidence to support this as a general practice is lacking. Additionally, placement of nasogastric tubes in their current form actually has an inherent risk of causing vomiting, which could counter-intuitively increase risk of aspiration. Furthermore, prolonged NG tube placement is a known risk factor for hospitalized patient morbidity. Thus, NG tube placement without appropriate indication (such as evidence of gastric distension) is inadvisable.

Histamine (H2) antagonists, such as cimetidine, famotidine, nizatidine, and ranitidine, and proton pump inhibitors (PPIs), such as dexlansoprazole, esomeprazole, lansoprazole, omeprazole, pantoprazole, and rabeprazole, have been shown to be effective in increasing the pH and reducing the volume of gastric contents. Prokinetics, such as domperidone, metoclopramide, erythromycin, and renzapride, promote gastric emptying and in turn should reduce the risk of aspiration. The efficacy of using such agents to prevent the sequelae of aspiration is likewise unsubstantiated.

US 2017/169725A discloses a system for monitoring stomach fullness which includes an ultrasound transponder configured to transmit ultrasound signals and receive reflected ultrasound signals. A processor is configured to process the received reflected ultrasound signals to determine level of stomach fullness and output fullness data indicative of the determined level of stomach fullness. A memory stores calibration data representative of calibrated levels of stomach fullness including empty and full. A stored program has executable codes to, upon execution, control the ultrasound transponder, compare the fullness data against the calibration data, and output alert data upon a match of a preset fullness level.

US 2019/142324A discloses a bladder volume detection device, the device comprising a sound transmitter and at least one receiver the device configured to ping the bladder with an acoustic signal having a resonant frequency of up to about 20 k Hz generated with said transmitter.

The present disclosure satisfies a long-felt need in the art of reducing the chance of pulmonary aspiration in patients at risk thereof.

### SUMMARY OF THE DISCLOSURE

The invention is defined in the independent claim. Further aspects and preferred embodiments are defined in the dependent claims. Any aspects, embodiments and examples of the present disclosure which do not fall under the scope of the appended claims do not form part of the invention and are merely provided for illustrative purposes.

Embodiments of the disclosure concern methods and systems for measuring gastric dimensions and utilizing such dimensions for the reduction in risk for pulmonary aspiration in an individual at risk thereof. The systems may comprise at least one energy generator and at least one energy detector. The energy generators may produce wave energy, including acoustic waves, mechanical waves, and/or electromagnetic waves. The energy detector(s) are capable of detecting the energy produced by the energy generator(s). In some embodiments, the energy detectors comprise a particular array of energy detectors. The array of energy detectors may be a one-dimensional array or a two-dimensional array. In some embodiments, the system comprises 2, 3, 4, 5, or more energy detectors. In some embodiments, the system comprises an attaching structure. The attaching structure may comprise a structure for attaching the energy detector(s) and/or the energy generator(s). The attaching structure may comprise means for securing the attaching structure to an individual. In some embodiments, the attaching structure comprises a means for securing the attaching structure to the skin of an individual. The attaching structure may comprise an adhesive of any kind, so long as the adhesive is not permanent.

Systems encompassed herein, and methods using such systems, may comprise a computing device. The computing device may be capable of processing data detected by any energy detector disclosed herein. The computing device may generate a gastric dimension from the data detected by the energy detectors.

Certain embodiments concern methods for reducing the risk of pulmonary aspiration in an individual. Certain embodiments concern methods for detecting gastric distension in an individual. In some embodiments, the method comprises placing any system encompassed herein on an individual in a suitable location, producing energy into the individual with an energy generator, and detecting the energy with an array of energy detectors on the individual. The detecting may generate refraction data, in specific embodiments. In some embodiments, the method comprises generating at least one source wave in the individual and detecting direct, reflected, and refracted waves from the source wave at one or more defined locations on the individual, wherein the detecting generates refraction data. The energy generator and/or energy detectors may be placed on an abdominal surface of the individual. The abdominal surface may be on a posterior, lateral, or anterior side of the individual. When there are multiple energy detectors, the array for the energy detectors may or may not be random. The detectors may be placed in a random or ordered pattern on the individual. In some embodiments, the pattern is a straight line, a triangle, a square, a circle, a diamond, a pentagon, a trapezoid, or a hexagon, merely as examples.

In some embodiments, the method comprises processing the refraction data to determine a gastric dimension of the individual. The individual may be administered at least one prophylactic and/or therapeutic interventions, including when the gastric dimension reaches or exceeds a threshold value indicative of the risk of pulmonary aspiration or gastric distension. In alternative cases, no action may be taken. In some embodiments, prophylactic and/or therapeutic interventions are administered to an individual when the gastric dimension comprises a gastric silhouette value more than about 10 cm, 11 cm, 12 cm, 13 cm, 14 cm, 15 cm, 16 cm, 17 cm, 18 cm, 19 cm, or 20 cm or more. In some embodiments, prophylactic and/or therapeutic interventions are administered to an individual when the gastric dimension comprises a gastric volume of more than about 500 cm³, 510 cm³, 525 cm³, 550 cm³, 575 cm³, 600 cm³, 625 cm³, 650 cm³, 675 cm³, 700 cm³ or more (Delgado-Aros et al., Gastroenterology 2004; 126:432-440; Delgado-Aros et al., Gastroenterology 2004; 127: 1685-1694). In some embodiments, prophylactic and/or therapeutic interventions are administered to an individual when the gastric dimension 100% increases from a baseline measurement in the individual (Delgado-Aros et al., Gastroenterology 2004; 126:432-440; Delgado-Aros et al., Gastroenterology 2004; 127: 1685-1694).

In some embodiments, an individual is administered a prophylactic and/or therapeutic intervention. The prophylactic and/or therapeutic intervention may comprise withdrawal of sedation, revised positioning of the individual, nasogastric tube placement, a histamine agonist, a proton pump inhibitor, a prokinetic, or a combination thereof. The histamine agonist may comprise cimetidine, famotidine, nizatidine, ranitidine, or a combination thereof. The proton pump inhibitor may comprise dexlansoprazole, esomeprazole, lansoprazole, omeprazole, pantoprazole, rabeprazole, or a combination thereof. The prokinetic may comprise domperidone, metoclopramide, erythromycin, renzapride, or a combination thereof.

Certain embodiments encompass detecting, diagnosing, treating, and/or monitoring an individual, including detecting gastric distension and detecting risks of pulmonary aspiration. In some embodiments, the individual's fasting state and/or gastric contents are unknown. In some embodiments, the individual has, or is suspected of having, delayed gastric emptying. The delayed gastric emptying may be caused by diabetic gastroparesis, advanced liver dysfunction, and/or advanced renal dysfunction. In some embodiments, the individual is critically ill. In some embodiments, the individual has unreliable or unclear medical history. In some cases, the individual has been non-compliant with instructions to maintain a fasting state.

It is contemplated that any embodiment discussed in this specification can be implemented with respect to any method, composition, and/or system of the disclosure, and vice versa. Furthermore, compositions and systems of the disclosure can be used to achieve methods of the disclosure.

Other objects, features and advantages of the present disclosure will become apparent from the following detailed description. It should be understood, however, that the detailed description and the specific examples, while indicating specific embodiments of the disclosure, are given by way of illustration only, since various changes and modifications within the scope of the disclosure will become apparent to those skilled in the art from this detailed description.

### BRIEF DESCRIPTION OF THE DRAWINGS

The following drawing forms part of the present specification and is included to further demonstrate certain aspects of the present disclosure. The disclosure may be better understood by reference the drawing in combination with the detailed description of specific embodiments presented herein.

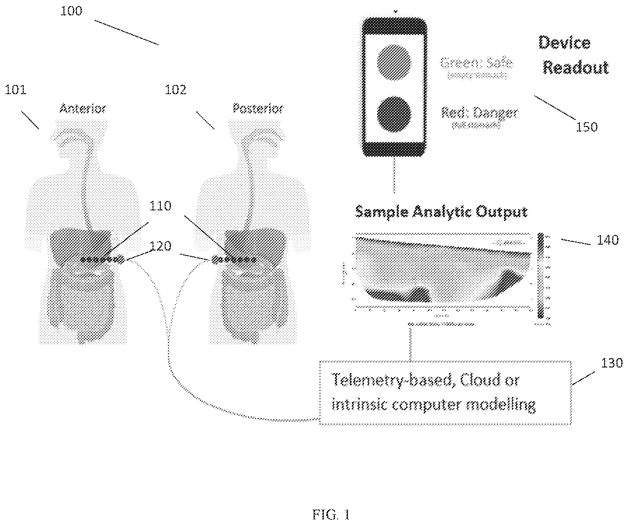

**FIG. 1** shows one embodiment of an array of receivers (depicted as smaller dark circles, merely as examples) and an acoustic generator (single larger circle) (or an alternative energy generator, including a mechanical energy generator) placed on an individual.

### DETAILED DESCRIPTION

### Definitions

Throughout this application, the term "about" is used to indicate that a value includes the inherent variation of error for the measurement or quantitation method.

The use of the word "a" or "an" when used in conjunction with the term "comprising" may mean "one," but it is also consistent with the meaning of "one or more," "at least one," and "one or more than one."

The phrase "and/or" means "and" or "or". To illustrate, A, B, and/or C includes: A alone, B alone, C alone, a combination of A and B, a combination of A and C, a combination of B and C, or a combination of A, B, and C. In other words, "and/or" operates as an inclusive or.

The words "comprising" (and any form of comprising, such as "comprise" and "comprises"), "having" (and any form of having, such as "have" and "has"), "including" (and any form of including, such as "includes" and "include") or "containing" (and any form of containing, such as "contains" and "contain") are inclusive or open-ended and do not exclude additional, unrecited elements or method steps.

The compositions and methods for their use can "comprise," "consist essentially of," or "consist of" any of the ingredients or steps disclosed throughout the specification. Compositions and methods "consisting essentially of" any of the ingredients or steps disclosed limits the scope of the claim to the specified materials or steps which do not materially affect the basic and novel characteristic of the claimed disclosure.

The term "gastric dimension" as used herein may refer to any size, length, width, diameter, radius, circumference, area, volume, capacity, proportion, or measurement of an individual's gastrointestinal tract or any organ of the gastrointestinal tract, including the stomach.

The term "gastric silhouette" may refer to the largest, smallest, or average length, diameter, radius, or circumference of the abdomen of an individual.

Reference throughout this specification to "one embodiment," "an embodiment," "a particular embodiment," "a related embodiment," "a certain embodiment," "an additional embodiment," or "a further embodiment" or combinations thereof means that a particular feature, structure or characteristic described in connection with the embodiment is included in at least one embodiment of the present disclosure. Thus, the appearances of the foregoing phrases in various places throughout this specification are not necessarily all referring to the same embodiment. Furthermore, the particular features, structures, or characteristics may be combined in any suitable manner in one or more embodiments.

A variety of aspects of this disclosure can be presented in a range format. It should be understood that the description in range format is merely for convenience and brevity and should not be construed as an inflexible limitation on the scope of the present disclosure. Accordingly, the description of a range should be considered to have specifically disclosed all the possible subranges as well as individual numerical values within that range as if explicitly written out. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 3, 4, 5, and 6. This applies regardless of the breadth of the range. When ranges are present, the ranges may include the range endpoints.

The term "individual" as used herein, which in some embodiments may be used interchangeably with the term "patient", generally refers to an individual in need of a therapy. The individual can be a mammal, such as a human, dog, cat, horse, pig or rodent. The individual can be a patient, e.g., have or be suspected of having or at risk for having a disease or medical condition. The individual may have a disease or be suspected of having the disease. The individual may be asymptomatic.

### Acoustic Refraction (AR) Methodology

Acoustic waves are mechanical perturbations that travel at a speed dictated by the acoustic impedance of the medium through which they travel. When an acoustic wave approaches an interface between two materials with different acoustic impedances, some of the wave energy will reflect off the interface, and some will refract through the interface. An array of acoustic receivers can be used to measure the time for the waves to travel back to the surface. The travel times from the source to various receivers and the velocity of the acoustic waves can then be used to reconstruct subsurface (tissue) interfaces with mathematical modeling techniques, including the Generalized Reciprocal Method (GRM) based on Snell's Law of refraction (3), to thereby outline the (gastric) volume circumscribed by delineated acoustic interfaces.

Certain embodiments of the disclosure encompass methods comprising generation and detection of energy in an individual. In some embodiments, the energy comprises a wave. The wave may be a mechanical wave, such as an acoustic wave. In some embodiments, the energy is electromagnetic energy. Any type of energy that generates waves capable of refraction may be used. Any frequency of wave may be used, including radio wave frequencies and/or ultrasonic frequencies. In some embodiments, one or more than one energy generator is used. In some embodiments, the wave travels through the individual from an energy generator. The wave may refract at tissue interfaces. In some embodiments, the direct wave (*i.e*. the unrefracted wave) and the refracted wave(s) are detected by one or more detectors. The detectors may comprise an array of detectors. The array of detectors may be a one-dimensional array or a two-dimensional array. The detectors may be arranged in any geometrical pattern, such as a line (including a straight line), a triangle, a square, a circle, a diamond, a pentagon, a trapezoid, or a hexagon. In some embodiments, there are 2, 3, 4, 5, 6, 7, 8, 9, 10, or more detectors. The detectors may be any suitable device capable of detecting the energy produced by the energy generator, such as an acoustic detector. The acoustic detector may be a microphone. In certain embodiments, refraction data is generated by measuring the time each acoustic detector detects the wave after the energy generator generates the wave.

Certain embodiments encompass methods for processing data, including refraction data produced by detection methods described herein. Any method capable of processing data may be used. In some embodiments, mathematical methods are used. In some embodiments, the mathematical model compares expected and actual detection times (*i.e.* the time each detector detects the wave generated by the energy generator). The mathematical method may comprise the generalized reciprocal method. The mathematical model may incorporate wave frequency, tissue-specific velocities, critical refraction angles, and other variables needed to establish one or more gastric dimensions. The mathematical method may comprise refractor velocity analysis and/or time-depth calculations.

As compared to the commonly used medical technique of ultrasound, which uses one sensor and one receiver unit that must be manually manipulated by a skilled operator, systems and methods encompassed herein may employ multiple spatially distanced (*i*.*e*, across a relatively large body surface area) receivers that in combination can collect data continually to map a two- or three-dimensional model, such as the abdomen. Importantly, as opposed to ultrasound, AR receivers used herein may be pre-placed in fixed positions to monitor the target image (*e.g.,* the stomach to assess gastric distension that is a risk marker for aspiration) and thus may not require a skilled operator to place, manipulate or analyze receiver-derived data. In some embodiments, the refraction data are obtained concurrently over any given area with any desired geometry. The refraction data may then be graphically represented and may be subjected to calculations without user input, making analysis easy and automated. In some embodiments, this capacity allows an automatic, user-independent signaling of abnormal gastric distension conditions, an essential risk signal for gastric aspiration. This signaling may trigger medical personnel to implement prophylactic aspiration-prevention actions, such as revised positioning, withdrawal of sedation, or NG tube placement. Adding to the beneficial ease and applicability of this approach, uniform receiver positions for assessing gastric size may be generically determined for all patients as determined by one skilled in the art, again eliminating the issue of user-specific knowledge or need for patient-specific receiver placement. In specific cases, the apparatus may be placed without the need for a specifically pre-determined location.

In comparison to the AR methods disclosed herein, ultrasound data acquisition, imaging and analysis rely heavily upon the user having knowledge of the anatomy and is limited by the depth of ultrasound penetration. For example, in order to map a 3-dimensional space such as the stomach, ultrasound scanners must be swept across the upper abdomen in a deliberate fashion so as to acquire gastric images. This requirement requires the user knowledge of the target (gastric) structure's anatomy, surrounding anatomy. Ultrasound-based imaging is also limited by air-filled and bony structures that restrict adequate data acquisition. The process by which ultrasound probe studies are conducted is also cumbersome in that the manual imaging process would need to be repeated on frequent occasions, each of which a new set of potentially inconsistent image sets would need to be acquired by a trained technician or practitioner.

Certain aspects of this disclosure concern applications of AR technology to be useful as a routine, low cost methodology to assess, including continually, the aspiration risk in essentially all clinical situations in which aspiration risk is a possibility, including prolonged, unclear or undetermined, as in hospitalized patients. In some embodiments, a gastric dimension is continually measured, measured at specific time periods, and/or measured when an individual arrives for a treatment. Any such measurement interval may establish a baseline measurement in the individual. Three specific additional clinical scenarios for AR use are: 1) pre-operative patients who have not followed fasting guidelines, either because of a communication gap, non-compliance, or because of the urgent nature of the clinical situation, 2) patients with delayed gastric emptying because of significant comorbidities in whom recommended fasting intervals may not reliably ensure an empty stomach (*e*.*g*. diabetic gastroparesis, advanced liver or renal dysfunction, critically ill patients), and 3) patients with unreliable or unclear history (*e*.*g*. language barrier, cognitive dysfunction, altered sensorium). The AR technology encompassed herein can assist clinicians in minimizing or eliminating aspiration risk in all such cases.

### Systems for Detecting Gastric Volume

Certain embodiments encompass systems for detecting gastric volumes. The systems may be capable of performing any method disclosed herein. In certain embodiments, the system generates mechanical waves or electromagnetic waves using an energy generator. The energy generator may comprise any device capable of producing mechanical waves or electromagnetic waves. In some embodiments, the energy generator produces percussive force. In some embodiments, the energy generator comprises a speaker. The energy generator may generate a wave at any frequency, including radio wave frequencies.

In certain embodiments, the system detects waves generated by an energy generator. In some embodiments, the system comprises one or more detectors. The detectors may comprise an array of detectors. The array of detectors may be a one dimensional array or a two dimensional array. The detectors may be arranged in a geometric pattern, such as a line (including a straight line), a triangle, a square, a circle, a diamond, a pentagon, a trapezoid, or a hexagon. The detectors may comprise any device capable of detecting energy, including wave energy. The detectors may comprise any device capable of detecting the energy produced by the energy generator. In some embodiments, the detector comprises a microphone. The detectors themselves may be of any shape and size. The detectors may be placed on the abdomen of the individual, such as below the diaphragm but above the pelvis. In some cases, the detectors may be placed on the back but corresponding to the region of the abdomen on the front. In specific embodiments where a three-dimensional output is desired, one or more detectors may be placed on the front of the individual and on the back of the individual.

In some embodiments, the system comprises an attaching structure for holding the energy generator and the detector(s). The attaching structure may comprise any suitable material for holding the energy generator and the detectors, such as plastic, metal, cloth, tape, or a combination thereof. In certain embodiments, the attaching structure comprises a means to secure the attaching structure to an individual. The attaching structure may comprise tape, straps, clips, belts, hooks, or other structures capable of securing the attaching structure to an individual. In some embodiments, the attaching structure comprises means to secure the attaching structure to the skin of an individual. The attaching structure may be capable of being secured to the individual's abdomen and/or back.

In some embodiments, the system comprises a computing device for processing data detected by the energy detectors. The computing device may use any method, including any mathematical method, described herein for computing or generating a gastric dimension.

Turning to FIG. 1, system **100** comprises (one or) multiple detectors **110** and energy generator **120** in either the anterior position **101** of the individual or the posterior position **102** of the individual. Data is collected from detectors **110** on the individual in the anterior position **101** or the posterior position **102** of the individual, uploaded to a local receiver **130,** such as a telemetry-based local receiver, or an encrypted cloud-based server that as a sample analytic output **140** analyzes data by simple computational algorithms based upon GRM calculations, or an artificial intelligence/neural network system, as needed. This algorithm is used to determine gastric distension based on acoustic refraction topographic modeling. A simple display readout **150,** such as a screen, provides the end-user with a message, indicating either a "safe" or "danger" recommendation regarding gastric risk assessment. In some cases, the readout is transferred electronically to a remote receiver, such as a cellular phone or tablet or computer. That is, in some cases a receiver/computer could be embedded in a patient-based device, and the readout may be sent remotely to signal an alarm.

### Individuals at Risk of Pulmonary Aspiration

Certain embodiments encompass detecting gastric distension, reducing the risk of pulmonary aspiration, and/or detecting a gastric dimension in an individual. In some embodiments, the individual is in need of emergency treatment, which may result in the individual's gastric contents being unknown. Other situations and circumstances may also be a reason for the individual's gastric contents to be unknown, such as a language barrier, mental incapacity, and/or refusal to communicate.

Methods and systems disclosed herein may be used to determine gastric contents of any individual, including individuals that are, or are soon to be, intubated, given anesthesia, unconscious, and/or asleep. In some embodiments, the individual has or has not fasted. The systems and methods disclosed herein may be used to determine whether an individual has or has not fasted. In some embodiments, the individual has, or is suspected of having, delayed gastric emptying. Delayed gastric emptying may be caused by a variety of diseases, syndromes, or illnesses such as diabetic gastroparesis, advanced liver dysfunction, and/or advanced renal dysfunction. In certain embodiments, the individual is critically ill or has unreliable or unclear medical history. The systems and methods disclosed herein may reduce the risk of placing an individual under anesthesia by measuring a gastric dimension of the individual and determining the individual's risk of pulmonary aspiration.

Certain embodiments encompass methods and systems for measuring one or more gastric dimensions in an individual. The measured gastric dimension may be used to determine whether the individual is at risk for pulmonary aspiration. Certain thresholds may be used to determine whether the individual is at risk for pulmonary aspiration and/or has gastric distension. In some embodiments, the threshold comprises a length of 1 cm, 2 cm, 3 cm, 4 cm, 5 cm, 6 cm, 7 cm, 8 cm, 9 cm, 10 cm, 11 cm, 12 cm, 13 cm, 14 cm, 15 cm, 16 cm, 17 cm, 18 cm, 19 cm, 20 cm, or more. The length may be the largest, smallest, or average length of an organ in the gastrointestinal tract, such as the stomach. In some embodiments, the threshold comprises a gastric volume of approximately 20 cm³, 21 cm³, 22 cm³, 23 cm³, 24 cm³, 25 cm³, 26 cm³, 27 cm³, 28 cm³, 29 cm³, 30 cm³, 31 cm³, 32 cm³, 33 cm³, 34 cm³, 35 cm³, 36 cm³, 37 cm³, 38 cm³, 39 cm³, 40 cm3, 41 cm³, 42 cm³, 43 cm³, 44 cm³, 45 cm³, 46 cm³, 47 cm³, 48 cm³, 49 cm³, 50 cm³, 55 cm³, 60 cm³, 65 cm³, 70 cm³, 75 cm³, 80 cm³, 85 cm³, 90 cm³, 95 cm³, 100 cm³, 110 cm³, 115 cm³, 120 cm³, 125 cm³, 130 cm³, 135 cm³, 140 cm³, 145 cm³, 150 cm³, 155 cm³, 160 cm³, 170 cm³, 175 cm³, 180 cm³, 185 cm³, 190 cm³, 195 cm³, 200 cm³, 210 cm³, 220 cm³, 230 cm³, 240 cm³, 250 cm³, 260 cm³, 270 cm³, 280 cm³, 290 cm³, 300 cm³, 325 cm³, 350 cm³, 375 cm³, 400 cm³, 425 cm³, 450 cm³, 475 cm³, 500 cm³, 525 cm³, 550 cm³, 575 cm³, 600 cm³, 625 cm³, 650 cm³, 675 cm³, 700 cm³, 725 cm³, 750 cm³, 775 cm³, 800 cm³, 825 cm³, 850 cm³, 875 cm³, 900 cm³, 925 cm³, 950 cm³, 975 cm³, 1000 cm³, 1100 cm³, 1200 cm³, 1300 cm³, 1400 cm³, 1500 cm³, or more. In some embodiments, the threshold comprises an increase in at least one gastric dimension from a previous measurement or from a known baseline. The increase may be approximately a 1%, 2%, 3%, 4%, 5%, 6%, 7%, 8%, 9%, 10%, 15%, 20%, 25%, 30%, 35%, 40%, 45%, 50%, 55%, 60%, 65%, 70%, 75%, 80%, 85%, 90%, 95%, 100%, 125%, 150%, 175%, 200%, 225%, 250%, 275%, 300%, 325%, 350%, 375%, 400%, 425%, 450%, 475%, 500%, 600%, 700%, 800%, 900%, 1000%, 1250%, 1500%, 1750%, 2000%, 2250%, 2500%, 2750%, 3000%, 3250%, 3500%, 3750%, 4000%, 4250%, 4500%, 4750%, 5000%, 6000%, 7000%, 8000%, 9000%, or more. The baseline of an individual may be determined by measuring a gastric dimension when the individual has known gastric contents or known fasting state. The baseline may be determined by measuring a gastric dimension one or more times. The baseline may be determined by continuously measuring a gastric dimension. In particular embodiments, the system allows for the ability of the operator to "dial in" any threshold setting (that is, any desired diameter or volume of any value).

### Examples

The following examples are included to demonstrate certain embodiments of the disclosure. It should be appreciated by those of skill in the art that the techniques disclosed in the example that follows represent techniques discovered by the inventors to function well in the practice of embodiments of the disclosure, and thus can be considered to constitute particular modes for its practice. However, those of skill in the art should, in light of the present disclosure, appreciate that many changes can be made in the specific embodiments that are disclosed and still obtain a like or similar result without departing from the scope of the disclosure.

### I. Example I: Solution for Preventative Detection of Gastric Distension Aspiration Risk

In one embodiment, the AR gastric imaging approach comprises an array of acoustic receivers adhered to the skin of a patient overlying their stomach and a mechanical and/or acoustic generator that is positioned in the middle of the receivers. The receivers detect the differing time of arrival of the acoustic energy waves generated by the generator at the different spatially distanced receiver points (FIG. 1). This array may or may not be adhered to the patient's abdomen with medical adhesive or medical tape either intermittently (e.g., at each eight-hour shift change) or for an extended duration to allow more frequent imaging analysis.

In one embodiment, the AR generator-receiver system transmits signal arrival times to an AR analysis computer that employs an algorithm, such as GRM calculations, to calculate gastric diameter. This computational element of the device, in certain embodiments, could be intrinsic to the device itself or it could be connected by radiofrequency or other telemetry methodologies, including for example to a mobile phone, remote telemetry cloud-based artificial intelligent/neural networks, or other suitable devices. The final output of this array may be as simple as a traffic light: red indicating a full stomach (exceeding normative gastric silhouette values) and therefore warranting further assessment or intervention, green indicating an empty stomach (falling within normative gastric silhouette values) with no further steps being indicated, and yellow indicating intermediate values and actions (these values could be pre-determined based on prospective studies and/or set by individual providers or institutions).

### * * *

All of the methods disclosed and claimed herein can be made and executed without undue experimentation in light of the present disclosure. While the compositions and methods of this disclosure have been described in terms of preferred embodiments, it will be apparent to those of skill in the art that variations may be applied to the methods and in the steps or in the sequence of steps of the method described herein without departing from the scope of the disclosure. More specifically, it will be apparent that certain agents which are both chemically and physiologically related may be substituted for the agents described herein while the same or similar results would be achieved. All such similar substitutes and modifications apparent to those skilled in the art are deemed to be within the scope of the disclosure as defined by the appended claims.

### REFERENCES

(1) Norma A. Metheny, PhD, Ray E. Clouse, MD, Yie-Hwa Chang, PhD, Barbara J. Stewart, PhD, Dana A. Oliver, MPH, and Marin H. Kollef, MD Tracheobronchial aspiration of gastric contents in critically ill tube-fed patients: frequency, outcomes, and risk factors. Crit Care Med. 2006; 34: 1007-1015.
(2) Lanspa MJ; Jones BE; Brown SM; Dean N. Mortality, morbidity, and disease severity of patients with aspiration pneumonia. J Hosp Med. 2013; 8: 83-90.
(3) Robert W. Lankston. The seismic refraction method: A viable tool for mapping shallow targets into the 1990s. Geophysics. 1989; 54: 1521-1663.

## Claims

1. A method for detecting gastric distention comprising generating at least one source wave on the abdomen of the individual and detecting direct, reflected and refracted waves from the source wave at one or more defined locations on the abdomen of the individual, **characterised in that** the method further comprising measuring the time each acoustic detector detects the wave after the energy generator generates the wave, generating refraction data using said times and detecting gastric distention in the individual based on the refraction data.

2. The method of claim 1, the method comprising the steps of:
a. placing a system on the abdomen of the individual comprising an energy generator and 2, 3, 4, 5, or more energy detectors, wherein the energy detectors are arranged in a one-dimensional or two-dimensional array;
b. producing mechanical waves into the abdomen of the individual with the energy generator; and
c. detecting the energy from the energy generator with the array of energy detectors, wherein the detecting generates refraction data.

3. The method of claim 1, further comprising determining a risk of pulmonary aspiration in the individual based on the refraction data.

4. The method of any one of claims 1-3, further comprising the step of processing the refraction data to determine:
a gastric dimension comprising any size, length, width, diameter, radius, circumference, area, volume, capacity, proportion, or measurement of the individual's stomach; and/or
a gastric silhouette comprising the average length, diameter, radius, or circumference of the individual's abdomen.

5. The method of claim 4, wherein the refraction data comprises gastric wall width and/or distance between the stomach and the abdomen.

6. The method of any one of claims 1-5, wherein the refraction data comprises automated measurements, repeated measurements, and/or a measurement change from baseline.

7. The method of any one of claims 1-6, wherein the method comprises processing detected waves by an artificial intelligence or neural network system to generate the refraction data.

8. The method of any one of claims 1-7, further comprising the step of generating a signal when the refraction data reaches or exceeds a threshold value indicative of a risk for pulmonary aspiration, and/or generating a signal without input from a skilled operator when the refraction data reaches or exceeds a threshold value indicative of a risk for pulmonary aspiration.

9. The method of any one of claims 1-8, wherein the system is placed in a fixed position on the abdomen of the individual throughout the producing mechanical waves and detecting steps.

10. The method of any one of claims 1-9, wherein the individual's fasting state and/or gastric contents are unknown.

11. The method of any one of claims 3-10, further comprising the step of determining a need for administering prophylactic and/or therapeutic interventions to the individual when the refraction data reaches or exceeds a threshold value indicative of the risk of pulmonary aspiration.

12. The method of any one of claims 1-11, wherein the detecting is continuous for eight hours or more.

13. The method of any one of claims 1-12, wherein the individual has or is suspected of having delayed gastric emptying caused by diabetic gastroparesis, advanced liver dysfunction, advanced renal dysfunction, medications, sepsis, inflammation, peritonitis, electrolyte abnormalities, and/or narcotics.

## Patentansprüche

1. Verfahren zum Erkennen einer Magenausdehnung, umfassend das Erzeugen mindestens einer Quellwelle am Abdomen der Person und das Erfassen direkter, reflektierter und gebrochener Wellen der Quellwelle an einer oder mehreren definierten Stellen am Abdomen der Person,
**dadurch gekennzeichnet, dass** das Verfahren ferner umfasst
Messen der Zeit, zu der jeder akustische Detektor die Welle erfasst, nachdem der Energiegenerator die Welle erzeugt hat, wodurch Brechungsdaten unter Verwendung der Zeiten erzeugt werden und eine Magenüberdehnung bei der Person basierend auf den Brechungsdaten erfasst wird.

2. Verfahren nach Anspruch 1, wobei das Verfahren die folgenden Schritte umfasst:
a. Anbringen eines Systems am Abdomen der Person, das einen Energiegenerator und 2, 3, 4, 5 oder mehr Energiedetektoren umfasst, wobei die Energiedetektoren in einer eindimensionalen oder zweidimensionalen Anordnung angeordnet sind;
b. Erzeugen mechanischer Wellen im Abdomen der Person mit dem Energiegenerator; und
c. Erfassen der Energie des Energiegenerators mit der Anordnung von Energiedetektoren, wobei durch das Erfassen Brechungsdaten erzeugt werden.

3. Verfahren nach Anspruch 1, das ferner das Bestimmen eines Risikos einer Lungenaspiration bei der Person basierend auf den Brechungsdaten umfasst.

4. Verfahren nach einem der Ansprüche 1-3, das ferner den Schritt der Verarbeitung der Brechungsdaten umfasst, um Folgendes zu bestimmen:
eine Magenabmessung, die eine beliebige Größe, Länge, Breite, einen Durchmesser, Radius, Umfang, Fläche, Volumen, Fassungsvermögen, Verhältnis oder Maß des Magens der Person umfasst; und/oder
eine Magensilhouette, die die durchschnittliche Länge, den Durchmesser, den Radius oder den Umfang des Abdomens der Person umfasst.

5. Verfahren nach Anspruch 4, wobei die Refraktionsdaten die Magenwandbreite und/oder den Abstand zwischen dem Magen und dem Abdomen umfassen.

6. Verfahren nach einem der Ansprüche 1-5, wobei die Refraktionsdaten automatisierte Messungen, wiederholte Messungen und/oder eine Messwertänderung gegenüber dem Ausgangswert umfassen.

7. Verfahren nach einem der Ansprüche 1-6, wobei das Verfahren die Verarbeitung der erfassten Wellen durch ein System mit künstlicher Intelligenz oder ein neuronales Netzwerk umfasst, um die Brechungsdaten zu erzeugen.

8. Verfahren nach einem der Ansprüche 1-7, das ferner den Schritt umfasst, ein Signal zu erzeugen, wenn die Brechungsdaten einen Schwellenwert erreichen oder überschreiten, der auf ein Risiko einer Lungenaspiration hinweist, und/oder ein Signal ohne Eingabe durch einen fachkundigen Bediener zu erzeugen, wenn die Refraktionsdaten einen Schwellenwert erreichen oder überschreiten, der auf ein Risiko einer Lungenaspiration hinweist.

9. Verfahren nach einem der Ansprüche 1-8, wobei das System während der gesamten Dauer der Erzeugung mechanischer Wellen und des Erfassens der Daten an einer festen Position auf dem Abdomen der Person angeordnet ist.

10. Verfahren nach einem der Ansprüche 1-9, wobei der Nüchternzustand und/oder der Mageninhalt der Person unbekannt sind.

11. Verfahren nach einem der Ansprüche 3-10, das ferner den Schritt des Bestimmens der Notwendigkeit umfasst, prophylaktische und/oder therapeutische Maßnahmen bei der Person durchzuführen, wenn die Refraktionsdaten einen Schwellenwert erreichen oder überschreiten, der auf das Risiko einer Lungenaspiration hinweist.

12. Verfahren nach einem der Ansprüche 1-11, wobei die Erfassung über acht Stunden oder länger kontinuierlich erfolgt.

13. Verfahren nach einem der Ansprüche 1-12, wobei die Person eine verzögerte Magenentleerung aufweist oder der Verdacht auf eine solche besteht, die durch diabetische Gastroparese, fortgeschrittene Leberfunktionsstörung, fortgeschrittene Nierenfunktionsstörung, Medikamente, Sepsis, Entzündung, Peritonitis, Elektrolytstörungen und/oder Betäubungsmittel verursacht wird.

## Revendications

1. Procédé de détection de distension gastrique comprenant la génération d'au moins une onde source sur l'abdomen de l'individu et la détection d'ondes directes, réfléchies et réfractées à partir de l'onde source en un ou plusieurs emplacements définis sur l'abdomen de l'individu, **caractérisé en ce que** le procédé comprend en outre la mesure du temps auquel chaque détecteur acoustique détecte l'onde après que le générateur d'énergie a généré l'onde, la génération de données de réfraction à l'aide desdits temps et la détection de distension gastrique chez l'individu sur la base des données de réfraction.

2. Procédé selon la revendication 1, le procédé comprenant les étapes consistant à :
a. placer un système sur l'abdomen de l'individu comprenant un générateur d'énergie et 2, 3, 4, 5 détecteurs d'énergie ou plus, dans lequel les détecteurs d'énergie sont agencés en réseau unidimensionnel ou bidimensionnel ;
b. produire des ondes mécaniques dans l'abdomen de l'individu avec le générateur d'énergie ; et
c. détecter l'énergie à partir du générateur d'énergie avec le réseau de détecteurs d'énergie, dans lequel la détection génère des données de réfraction.

3. Procédé selon la revendication 1, comprenant en outre la détermination d'un risque d'aspiration pulmonaire chez l'individu sur la base des données de réfraction.

4. Procédé selon l'une quelconque des revendications 1 à 3, comprenant en outre l'étape consistant à traiter les données de réfraction pour déterminer :
une dimension gastrique comprenant une taille, une longueur, une largeur, un diamètre, un rayon, une circonférence, une aire, un volume, une capacité, une proportion, ou une mesure de l'estomac de l'individu ; et/ou
une silhouette gastrique comprenant une moyenne de longueur, de diamètre, de rayon, ou de circonférence de l'abdomen de l'individu.

5. Procédé selon la revendication 4, dans lequel les données de réfraction comprennent une largeur de paroi gastrique et/ou une distance entre l'estomac et l'abdomen.

6. Procédé selon l'une quelconque des revendications 1 à 5, dans lequel les données de réfraction comprennent des mesures automatisées, des mesures répétées, et/ou un changement de mesure par rapport à la ligne de base.

7. Procédé selon l'une quelconque des revendications 1 à 6, dans lequel le procédé comprend le traitement d'ondes détectées par un système d'intelligence artificielle ou de réseau neuronal pour générer les données de réfraction.

8. Procédé selon l'une quelconque des revendications 1 à 7, comprenant en outre l'étape consistant à générer un signal lorsque les données de réfraction atteignent ou dépassent une valeur seuil indicative d'un risque d'aspiration pulmonaire, et/ou générer un signal sans entrée à partir d'un opérateur compétent lorsque les données de réfraction atteignent ou dépassent une valeur seuil indicative d'un risque d'aspiration pulmonaire.

9. Procédé selon l'une quelconque des revendications 1 à 8, dans le système est placé dans une position fixe sur l'abdomen de l'individu tout au long des étapes de production d'ondes mécaniques et détection.

10. Procédé selon l'une quelconque des revendications 1 à 9, dans lequel l'état à jeun et/ou le contenu gastrique de l'individu sont inconnus.

11. Procédé selon l'une quelconque des revendications 3 à 10, comprenant en outre l'étape consistant à déterminer une nécessité d'administrer des interventions prophylactiques et/ou thérapeutique à l'individu lorsque les données de réfraction atteignent ou dépassent une valeur seuil indicative du risque d'aspiration pulmonaire.

12. Procédé selon l'une quelconque des revendications 1 à 11, dans lequel la détection est continue pendant huit heures ou plus.

13. Procédé selon l'une quelconque des revendications 1 à 12, dans lequel l'individu a ou est soupçonné d'avoir une vidange gastrique retardée causée par une gastroparésie diabétique, une insuffisance hépatique avancée, une insuffisance rénale avancée, des médications, une septicémie, une inflammation, une péritonite, des anomalies des électrolytes, et/ou l'usage de narcotiques.
